# EUROPÄISCHE PATENTSCHRIFT

(11) **EP 2 892 471 B1**
(45) Veröffentlichungstag und Bekanntmachung des Hinweises auf die Patenterteilung: **06.05.2020**
(21) Anmeldenummer: 13758753.1
(22) Anmeldetag: 03.09.2013
(51) Int. Cl.: A61F 2/78, A61F 2/80

(54) **LINER FÜR EINE PROTHESE UND PROTHESE**
LINER FOR A PROSTHESIS, AND PROSTHESIS
MANCHON POUR PROTHÈSE ET PROTHÈSE

(30) Priorität: 03.09.2012 DE 102012017324
(43) Veröffentlichungstag der Anmeldung: 15.07.2015
(73) Patentinhaber: Ottobock SE & Co. KGaA, 37115 Duderstadt (DE)
(72) Erfinder: MOSLER, Lüder, 37115 Duderstadt (DE); MÜLLER, Christian, 37547 Kreiensen (DE); KETTWIG, Thomas, 37085 Göttingen (DE)
(74) Vertreter: Friedrich, Andreas
(86) Internationale Anmeldenummer: PCT/EP2013/002636
(87) Internationale Veröffentlichungsnummer: WO 2014/032815

(56) Entgegenhaltungen:
- WO-A1-98/04218
- GB-A- 826 041
- US-A- 5 507 722
- US-A1- 2007 043 450
- US-B1- 6 440 172

## Beschreibung

Die Erfindung betrifft einen Liner für eine Prothese, der eine proximale Öffnung zum Aufnehmen eines Amputationsstumpfes und ein distales Ende aufweist und der aus einem Linermaterial hergestellt ist, wobei sich eine Längsrichtung des Liners von der proximalen Öffnung zu dem distalen Ende erstreckt und eine Umfangsrichtung senkrecht zu der Längsrichtung verläuft. Die Erfindung betrifft zudem eine Prothese mit einem derartigen Liner.

Gattungsgemäße Liner und mit ihnen ausgestattete Prothesen sind seit langer Zeit aus dem Stand der Technik bekannt. Die Prothese verfügt über einen Prothesenschaft, der herkömmlicherweise aus einem starren Material, beispielsweise einem faserverstärkten Kunststoff, hergestellt wurde. Dieses Material ist leicht zu verarbeiten, stabil und weist dennoch ein geringes Eigengewicht auf, so dass es für Bauteile einer Prothese sehr geeignet ist. Um den Kontakt zwischen der Haut des Amputationsstumpfes des Patienten und dem starren Prothesenschaft zu vermeiden und gleichzeitig für eine Polsterung zu sorgen, wird über den Amputationsstumpf zunächst ein Liner aus einem Linermaterial gezogen, bevor der Amputationsstumpf in den Prothesenschaft eingeführt wird. Ein geeignetes Linermaterial ist beispielsweise Silikon. Das Linermaterial schmiegt sich dabei an die Form des Amputationsstumpfes an und verfügt daher über eine optimale Passform und sorgt für einen optimalen Sitz.

Die Prothese verfügt zudem über eine Protheseneinrichtung, die am Prothesenschaft angeordnet ist und unter anderem eine künstliche Gliedmaße, beispielsweise einen künstlichen Unterschenkel mit einem daran angeordneten Fuß umfasst. Teile der Protheseneinrichtung sind zudem Befestigungs- und Arretierungsmittel, sowie gegebenenfalls Pumpeinrichtungen, Ventile und ähnliches, was zum Betrieb der Prothese benötigt wird.

An dem Liner wird anschließend der Prothesenschaft angeordnet. Herkömmlicherweise besteht der Prothesenschaft vollflächig aus dem starren Material. Der Amputationsstumpf befindet sich folglich in einer Hülle, die ihn vollständig von der Umgebung trennt. Insbesondere ist es für den Träger der Prothese nicht möglich, über den Amputationsstumpf Eindrücke der Umwelt, beispielsweise Berührungen am Amputationsstumpf, wahrzunehmen. Zudem wird durch die vollflächige Ummantelung des Liners, der sich am Amputationsstumpf befindet, die Atmungsaktivität des Linermateriales deutlich herabgesetzt und eingeschränkt. Beides führt zu einem unangenehmen und künstlichen Tragegefühl und zu einem verminderten Tragekomfort der Prothese und des Liners.

Insbesondere bei Beinprothesen weisen Rahmenschäfte, die an wenigstens einer Stelle durchbrochen sind und somit wenigstens ein Fenster aufweisen, jedoch den Nachteil auf, dass es aufgrund der sehr hohen Belastung des Amputationsstumpfes beim Gehen mit der Prothese zu einem Herausquellen des mit dem Liner überzogenen Amputationsstumpfes aus den Fenstern des Rahmenschaftes kommen kann. Dadurch kann es zu Druckstellen und durch Scheuern insbesondere an den Rändern der Fenster des Rahmenschaftes auch zu teilweise schmerzhaften Wunden, insbesondere in Form so genannter Fensterödeme, kommen.

Zudem ist es bei einem Rahmenschaft naturgemäß nicht möglich, den Rahmenschaft an dem Amputationsstumpf bzw. den darüber gezogenen Liner festzulegen, indem zwischen dem Liner und dem Liner Prothesenschaft ein Unterdruck erzeugt wird. Da der Rahmenschaft wenigstens eine Durchbrechung in Form des Fensters aufweist, existiert hier kein abgeschlossenes Volumen, das mit einem Unterdruck beaufschlagt werden könnte. Daher werden Rahmenschäfte an Linern herkömmlicherweise über eine am distalen Ende des Liners vorgesehene Befestigungseinrichtung befestigt. Dadurch wirken die insbesondere in der Schwungphase des Gehens mit der Prothese auftretenden Zugkräfte ausschließlich auf das distale Ende des Liners, wodurch es zu einem so genannten Melkeffekt kommen kann. Dieser führt im Laufe der Zeit, beispielsweise beim Tragen der Prothese über einen ganzen Tag, zu einer Volumenänderung und einer Formänderung des Amputationsstumpfes. Das Linermaterial muss flexibel genug sein, um dieser Form- und Volumenänderung des Amputationsstumpfes folgen zu können, um einen festen Sitz der Prothese am Amputationsstumpf des Patienten zu gewährleisten. Durch ein dafür benötigtes besonders flexibles Linermaterial wird jedoch ein Herausquellen des Liners bzw. des mit ihm überzogenen Amputationsstumpfes aus den Fenstern des Rahmenschaftes erleichtert.

Aus der US 6,440,172 B1 ist ein gattungsgemäßer Liner bekannt, der aus einem elastischen Material besteht. Dabei handelt es sich um einen textilen Liner, der zwischen einem Gelliner und einem Prothesenschaft getragen werden soll. Ein ähnlicher Liner ist auch aus der US 2007/0043450 A1 bekannt.

Die GB 826,041 beschreibt einen Liner, der entweder aus einem elastischen Gewebe oder aus Silikon oder einem anderen synthetischen elastischen Material bestehen kann. Ein ähnlicher Liner aus Nylon oder Elastomermaterialien wird in der WO 98/04218 A1 beschrieben.
Der Erfindung liegt somit die Aufgabe zugrunde, einen Liner und eine damit ausgerüstete Prothese vorzuschlagen, mit der ein sicherer Halt des Liners und der Prothese am Amputationsstumpf des Patienten auch über einen langen Zeitraum hinweg gewährleistet ist, und mit der gleichzeitig ein Herausquellen des Amputationsstumpfes aus den Fenstern eines Rahmenschaftes wirksam verhindert wird.

Die Erfindung löst die gestellte Aufgabe durch einen gattungsgemäßen Liner gemäß dem Oberbegriff von Anspruch 1, der sich dadurch auszeichnet, dass der Liner eine Mehrzahl von Fasern aufweist, die aus einem unelastischen Material bestehen und derart angeordnet sind, dass eine Verlängerung des Liners in Längsrichtung eine Verkürzung des Liners in Umfangsrichtung und umgekehrt zur Folge hat, wobei der Liner rutschhemmende Mittel aufweist, durch die ein Verrutschen des Liners an dem Amputationsstumpf in einem angelegten Zustand des Liners verhindert wird.

Vorteilhafterweise sind die Fasern dabei so angeordnet, dass eine elastische Verformung des Liners sowohl in Längsrichtung als auch in Umfangsrichtung möglich ist. Dies ist jedoch nicht zwangsläufig der Fall. Wichtig ist lediglich, dass der Liner beim Anziehen des Liners, also wenn der Amputationsstumpf in den Liner eingebracht wird, sich optimal der Form des Amputationsstumpfes anpassen kann. Dies geschieht in der Regel, indem der Liner vom distalen Ende des Amputationsstumpfes an aufgerollt und so großflächig mit dem Amputationsstumpf in Kontakt gebracht wird.

Vorteilhafterweise sind dabei die Fasern in dem Linermaterial eingebettet. Dies hat zur Folge, dass es keine störenden Druckstellen oder Unebenheiten am Liner gibt, die zu Unbequemlichkeiten oder sogar Schmerzen führen können.

Der Liner kann sich folglich sowohl in Umfangsrichtung als auch in Längsrichtung optimal an die jeweilige Kontur des Amputationsstumpfes anpassen, insbesondere wenn in beiden Richtungen eine elastische Verformung möglich ist. Im Laufe eines Tages ändert sich das Volumen des Amputationsstumpfes. Nimmt sein Umfang beispielsweise ab, müsste auch der Liner in Umfangsrichtung kürzer werden um weiterhin einen optimalen Sitz zu gewährleisten. Das hätte jedoch eine Verlängerung des Liners in Längsrichtung zur Folge. Das bedeutet, dass sich ein großer Anteil der Innenfläche des Liners, die mit der Haut des Trägers in Kontakt ist, relativ zu der Haut verschieben müsste. Dies ist aufgrund der rutschhemmenden Mittel jedoch nicht möglich, so dass der Liner der Volumenänderung nicht folgt. Dies ist insbesondere dann von Vorteil, wenn das Volumen des Amputationsstumpfes stark zunimmt und es so zu einem Herausquellen des Stumpfes aus Fenstern des Prothesenschaftes kommen kann. Der Liner müsste sich in Umfangsrichtung erweitern, was jedoch eine Verkürzung in Längsrichtung zur Folge hätte und daher aufgrund der rutschhemmenden Mittel nicht möglich ist.

Soll der Liner an das geänderte Volumen des Amputationsstumpfes angepasst werden, muss der Liner lediglich vom Amputationsstumpf entfernt und erneut über ihn gezogen werden. In diesem Fall kann sich der Liner erneut optimal der nun vorliegenden Form des Amputationsstumpfes anpassen.

Eine elastische Verformung des Liners in einer Richtung hat folglich immer eine Verformung in der anderen Richtung zur Folge, die mit einer großflächigen Verschiebung des Linermaterials relativ zum Amputationsstumpf des Patienten einher geht und daher im angelegten Zustand des Liners nicht möglich ist.

In einer bevorzugten Ausführungsform sind die rutschhemmenden Mittel eine Oberflächenbeschaffenheit der Innenseite des Liners, die in dem angelegten Zustand des Liners an dem Amputationsstumpf anliegt. Es kommt aufgrund dieser Oberflächenbeschaffenheit zu einer erhöhten Haftreibung bzw. Haftung zwischen der Innenfläche des Liners und der Haut des Trägers des Liners, so dass in diesem Fall aufgrund der erhöhten Haftreibung bzw. Haftung ein großflächiges Verschieben und Verrutschen des Lines am Amputationsstumpf, wie es für eine Volumenänderung nötig wäre, nicht stattfinden kann.

In einer bevorzugten Ausgestaltung des Liners ist seine Innenfläche, die im angelegten Zustand mit der Haut des Trägers der Prothese in Kontakt kommt, mit einer Beschichtung versehen, die eine Haftreibung zwischen dem Liner und der Haut des Trägers verbessert und so den gewünschten Effekt verstärkt. Alternativ oder zusätzlich dazu kann auch ein separater Zusatzliner vorgesehen sind, der über den eigentlichen Liner gezogen wird und der eine erhöhte Stabilität in Längsrichtung aufweist, um Kräfte in Längsrichtung aufnehmen zu können.

Alternativ oder zusätzlich dazu können die rutschhemmenden Mittel wenigstens ein Umschließungselement aufweisen, das eingerichtet ist, den Amputationsstumpf im angelegten Zustand so zu umschließen, dass ein Verrutschen des Liners verhindert wird. Das Umschließungselement kann dabei beispielsweise ein socken- oder schlauchartiges Element sein, das nach dem Anordnen des Liners am Amputationsstumpf über den Liner gezogen wird und beispielsweise im proximalen Bereich des Liners angeordnet wird. Das Umschließungselement übt dabei über den Umfang des Amputationsstumpfes, an dem sich der Liner befindet, einen Druck auf den Liner aus und sorgt so dafür, dass in diesem Bereich eine erhöhte Haftreibung zwischen der Innenfläche des Lines und dem Amputationsstumpf zustande kommt. Auch auf diese Weise ist es beispielsweise ohne eine Oberflächenbeschichtung der Innenseite des Liners möglich, die benötigte Haftreibung zu erreichen, um ein Verrutschen des Lines an dem Amputationsstumpf zu verhindern. Abhängig von der benötigten Stärke der Haftreibung und der bereits durch die bereits auf anderem Wege erreichte Haftreibung kann die Breite des Umschließungselementes in Längsrichtung des Lines variieren. Wird lediglich eine geringe Erhöhung der Haftreibung durch das Umschließungselement benötigt, kann es entsprechend schmal ausgebildet werden, während es breit ausgebildet werden sollte, wenn eine starke Erhöhung der Haftreibung zwischen der Innenseite des Lines und dem Amputationsstumpf erreicht werden soll.

Das Umschließungselement muss dabei den Amputationsstumpf nicht vollständig, also um den gesamten Umfang, umschließen. Auch eine teilweise Umschließung kann ausreichend sein, um die nötigen Kräfte auf den Liner und den Amputationsstumpf aufzubringen.
Das Umschließungselement kann auch in Form beispielsweise eines Kunststoffbauteils, beispielsweise aus einem Plastik, bestehen, das über zwei laschenartige Vorsprünge verfügt, die um den Amputationsstumpf herumgelegt werden. Über ein Befestigungselement sind die beiden laschenartigen Vorsprünge miteinander verbindbar, wobei auf diese Weise der zum Verhindern des Verrutschens des Liners nötige Druck aufgebracht wird. Insbesondere lässt sich vorteilhafterweise der durch die beiden laschenartigen Vorsprünge eingeschlossene Umfang stufenlos einstellen, so dass auch die auf den Amputationsstumpf aufgebrachte Kraft optimal an die Bedürfnisse des Trägers angepasst werden kann. Zum Verschließen kann beispielsweise ein band- oder gurtartiges Element an einer der beiden Laschen angeordnet sein, das beispielsweise über Klettverschlusselemente an dem jeweils anderen laschenartigen Vorsprung befestigbar ist. Möglich ist natürlich auch, dass das Element durch eine Schlaufe gezogen und ebenfalls über Klettverschlusselemente an sich selbst befestigt werden kann.

In einer anderen Ausgestaltung ist das Kunststoffelement so ausgebildet, dass es beim Anlegen der Prothese an dem Amputationsstumpf aufgeweitet und so elastisch vorgespannt wird. Dadurch wird eine Klemmkraft auf den an dem Amputationsstumpf angeordneten Liner aufgebracht, die ebenfalls ein Verrutschen des Liners am Stumpf verhindert.

Vorteilhafterweise weist die Mehrzahl von Fasern wenigstens eine erste Faser und wenigstens eine zweite Faser auf, die derart angeordnet sind, dass sie sich gegenseitig vorzugsweise unter einem rechten Winkel kreuzen. Dies bezieht sich auf den Liner im in keiner Richtung gedehnten oder elastisch verformten Zustand. Als besonders vorteilhaft hat sich herausgestellt, wenn die erste Faser und die zweite Faser ein Rautenmuster bilden, bei dem die Innenwinkel jeder Raute rechte Winkel sind und die Fasern einen Winkel von etwa 45° beziehungsweise -45° mit der Längsrichtung des Liners einschließen.

Dadurch, dass in dem Linermaterial wenigstens eine erste Faser und wenigstens eine zweite Faser vorgesehen sind, die derartig angeordnet sind, dass sie einander gegenseitig kreuzen, entsteht ein volumenstabiler Liner. Wird ein erfindungsgemäßer Liner über einen Amputationsstrumpf gezogen, passt er sich aufgrund der Flexibilität und Elastizität des Linermateriales optimal an den Amputationsstumpf an. Herkömmlicherweise wird ein derartiger Liner auf dem Amputationsstumpf abgerollt, so dass das Anlegen des erfindungsgemäßen Liners wie bei einem herkömmlichen Liner erfolgen kann. Die in dem Linermaterial eingebetteten ersten Fasern und zweiten Fasern ordnen sich dabei so an, dass sie optimal der Form des Amputationsstumpfes folgen. Beim Anlegen des Liners an den Amputationsstumpf folgen auch die wenigstens eine erste Faser und die wenigstens eine zweite Faser der Außenkontur des Amputationsstumpfes, so dass sich ein Winkel, unter dem sich die beiden Fasern kreuzen, frei einstellt. Grenzen dieser Einstellbarkeit des Winkels sind nur durch das Linermaterial gegeben, in dem die ersten Fasern und die zweiten Fasern eingebettet sind.

In einer bevorzugten Ausführungsform bildet die erste Faser eine spiralförmige Matrix in einer ersten Umlaufrichtung und die zweite Faser eine spiralförmige Matrix in einer der ersten Umlaufrichtung entgegengesetzten zweiten Umlaufrichtung. Spiralförmig bedeutet in diesem Fall, dass sich die jeweilige Faser vorzugsweise in mehreren Windungen um den Umfang des Amputationsstumpfes herum erstreckt, wenn der Liner an einem Amputationsstumpf anliegt, und dabei beispielsweise von dem distalen Ende des Liners zu der proximalen Öffnung verläuft. Dadurch, dass sich die erste Faser und die zweite Faser in unterschiedlichen Umlaufrichtungen um den Amputationsstumpf herum erstrecken, wenn der Liner angelegt ist, kommt es zu einem Kreuzen der beiden Fasern an einer Vielzahl unterschiedlicher Stellen. Damit ist die gewünschte Volumenstabilität erreicht und gleichzeitig eine optimale Formanpassung des Liners beim Anlegen an den Amputationsstumpf gewährleistet.

Vorzugsweise ist die erste Faser Teil einer ersten Faserlage aus einer Mehrzahl von ersten Fasern und die zweite Faser ist Teil einer zweiten Faserlage aus einer Mehrzahl zweiter Fasern. Die einzelnen Fasern jeder Faserlage können dabei vorzugsweise zu einander parallel verlaufen. In dieser Ausgestaltung können großflächige Faserlagen verwendet werden, die für den jeweils gewünschten Liner auf die optimale Größe zugeschnitten werden können.

Bevorzugt weist der Liner wenigstens zwei Teilliner auf, wobei die wenigstens eine erste Faser in einem ersten Teilliner und die wenigstens eine zweite Faser in einem zweiten Teilliner eingebettet ist und der zweite Teilliner vorgesehen ist, über einen Amputationsstumpf gezogen zu werden, nachdem über den Amputationsstumpf der erste Teilliner gezogen wurde. Die wenigstens zwei Teilliner sind folglich beide ausgebildet, um über den Amputationsstumpf gezogen zu werden. Sie bestehen jeweils aus einem Linermaterial, in das jeweils nur eine der vorgesehenen Faserarten eingebettet ist. Durch ein Übereinanderziehen der wenigstens zwei Teilliner kommt es zu der gewünschten Überlagerung der wenigstens zwei Fasern derart, dass sie sich unter einem Winkel kreuzen. An diesem Beispiel wird besonders deutlich, dass es im Rahmen der vorliegenden Erfindung nicht nötig ist, dass sich die unterschiedlichen Fasern beim Kreuzen berühren. Wichtig ist lediglich, dass sie in unterschiedliche Richtungen verlaufen und dass sie eine elastische Verformung des Liners in Längsrichtung und in Umfangsrichtung erlauben. Ein Kreuzen kann auch in einem gewissen Abstand voneinander geschehen.

In einer anderen vorteilhaften Ausgestaltung erstrecken sich die Fasern in Längsrichtung des Liners von dem distalen Ende zu der proximalen Öffnung. Dies hat zur Folge, dass eine elastische Verlängerung des Liners in dieser Richtung nicht stattfinden kann. Dennoch ist auch ein solcher Liner in der Lage, sich beim Abrollen auf einem Amputationsstumpf an unterschiedlichste Volumen und Ausdehnungen des jeweiligen Amputationsstumpfes anzupassen und den Amputationsstumpf sicher zu umfassen. Ist ein solcher Liner auf einen schmalen Amputationsstumpf aufgerollt worden, der folglich nur einen geringen Umfang aufweist, befindet sich der proximale Rand, der die proximale Öffnung des Liners umgibt, am Amputationsstumpf weiter oben, als wenn derselbe Liner über einen dicken Amputationsstumpf, der folglich einen großen Umfang aufweist, gezogen würde. Auch in diesem Fall hat eine Veränderung des Amputationsstumpfvolumens, beispielsweise eine Zunahme des Volumens, zur Folge, dass der Liner dieser Volumenänderung nur folgen kann, wenn der proximale Rand des Liners am Amputationsstumpf des Patienten verrutscht. Dies ist jedoch mit einem großflächigen Verrutschen der gesamten Innenseite des Liners am Amputationsstumpf verbunden, was durch die rutschhemmenden Mittel verhindert wird.

Alternativ ist es auch möglich, dass die Mehrzahl von Fasern eine netzartige Struktur bildet, die eine Mehrzahl aneinander angrenzender Maschen aufweist. Vorzugsweise haben die Maschen eine hexagonale Form. Derartige Netze oder netzartige Strukturen haben die erforderlichen Eigenschaften und ermöglichen eine elastische Verformung in Längsrichtung und in Umfangsrichtung des Liners, sofern sie in der richtigen Orientierung angeordnet sind. Auch diese Netze können großflächig vorgefertigt werden und bei der Herstellung der Liner zugeschnitten und in dem Linermaterial eingebettet werden. Dadurch wird das Herstellungsverfahren vereinfacht und verschlankt und somit die Produktionskosten gesenkt.

Eine erfindungsgemäße Prothese verfügt über einen oben beschriebenen Liner, einen Prothesenschaft und eine distale Protheseneinrichtung, die an einem distalen Ende des Prothesenschaftes festlegbar ist. Dabei verfügt der Prothesenschaft vorzugsweise über einen proximalen Schaftbereich und einen distalen Schaftbereich, die durch eine Verbindungseinrichtung voneinander beabstandet miteinander verbunden sind.

Der Prothesenschaft ist folglich als Rahmenschaft ausgebildet, wodurch er die bereits genannten Vorteile aufweist. Der distale Schaftbereich ist vorteilhafterweise in Form einer Schale oder Tasse ausgebildet, und eingerichtet, um das distale Ende des Amputationsstumpfes aufzunehmen. Über den proximalen Schaftbereich wird der Prothesenschaft am Amputationsstumpf festgelegt und in einer bestimmten Ausrichtung fixiert. Auf diese Weise erhält der Patient eine ausreichend stabilisierte Verbindung zur Prothese und die Möglichkeit, diese in optimaler Weise zu steuern. Eine derartige Prothese ist insbesondere eine Transtibialprothese. Eine derartige Prothese wird verwendet, wenn ein Bein eines Patienten unterhalb des Knies amputiert werden musste. Der dem Patienten verbliebene Rest des Unterschenkels ist dabei von Patient zu Patient stark unterschiedlich lang. Durch die Verbindungseinrichtung, mit der der proximale Schaftbereich mit dem distalen Schaftbereich verbunden ist, kann auf diese Länge eingegangen werden, indem die Verbindungseinrichtung in ihrer Länge verstellbar ausgebildet ist. Je länger die Verbindungseinrichtung ausgebildet werden kann, desto weiter sind der proximale Schaftbereich und der distale Schaftbereich voneinander getrennt und desto größer sind die Kontrolle und die Steuerbarkeit der Prothese durch den Patienten. Gleichzeitig werden auf diese Weise die Durchbrüche in dem derart ausgebildeten Rahmenschaft möglichst groß ausgebildet, so dass deren Vorteile möglichst umfassend zum Tragen kommen.

Der Prothesenschaft kann am Liner in verschiedenen Formen festgelegt werden. Beispielsweise kann an einem distalen Ende des Liners eine Befestigungseinrichtung, z. B. in Form eines nach distal hervorstehenden Pins vorgesehen sein, die in eine dafür vorgesehene Aufnahmeeinrichtung am Prothesenschaft eingesetzt werden kann. Alternativ kann auch über eine Flächen- oder Mantelreibung zwischen der Außenseite des Linermaterials und der Innenseite des Prothesenschaftes eine Haltekraft zwischen dem Prothesenschaft und dem Liner hervorgerufen werden. Bevorzugt werden beide Effekte miteinander kombiniert, um einen möglichst sicheren Halt des Prothesenschaftes am Liner und damit auch an dem Amputationsstumpf zu gewährleisten.
In einer bevorzugten Ausführungsform weist der proximale Schaftbereich eine Öffnung zum Aufnehmen des Amputationsstumpfes auf, wobei eine Umfangslänge der Öffnung einstellbar ist. Dies kann beispielsweise durch wenigstens zwei Schaftelemente erreicht werden, die über wenigstens ein Verstellelement zueinander verstellbar sind. Damit wird gewährleistet, dass die Größe der Öffnung, in die der Amputationsstumpf mit dem darüber gezogenen Liner eingebracht wird, individuell einstellbar ist. Dadurch kann ein Standardschaft verwendet werden, der an die individuellen Gegebenheiten des Amputationsstumpfes des Patienten anpassbar ist. Die beiden Schaftelemente können beispielsweise in Form von zungenförmigen Vorsprüngen ausgebildet sein, die in Umfangsrichtung aufeinander zu gerichtet sind. Über einen Gurt oder eine andere geeignete Verbindungseinrichtung können diese Vorsprünge miteinander verbunden werden, wobei der Gurt vorzugsweise in seiner Länge veränderbar ist. So kann der Umfang der proximalen Öffnung des Schaftes geändert und an die individuellen Gegebenheiten des Patienten angepasst werden.

Insbesondere ist es durch die gewählte Ausgestaltung der Prothese möglich, unterschiedliche Standardbauteile miteinander zu kombinieren und so eine jeweils individuell angepasste Prothese zu erreichen. So ist es beispielsweise möglich, unterschiedliche distale Schaftbereiche, also beispielsweise Tassen unterschiedlicher Größe und Tiefe, sowie unterschiedliche proximale Schaftbereiche und unterschiedlich lange Verbindungseinrichtungen zu bevorraten, die jeweils untereinander kombinierbar ausgebildet sind. Eine individuelle Anpassung an den Amputationsstumpf des Patienten wird so ermöglicht.

An dem distalen und/oder proximalen Ende des Prothesenschaftes ist eine Protheseneinrichtung festlegbar, die beispielsweise in Form eines künstlichen Unterschenkels mit einem sich daran befindlichen Prothesenfuß bestehen kann. Auch diese Protheseneinrichtung kann in unterschiedlichste Richtungen verschwenkbar ausgebildet sein, um die optimale Position für den jeweiligen Amputationsstumpf des Patienten zu erreichen. Gleichzeitig ist insbesondere der künstliche Unterschenkel längenverstellbar, um auf unterschiedlich lange Amputationsstümpfe reagieren zu können.

Mit Hilfe einer Zeichnung wird nachfolgend ein Ausführungsbeispiel der vorliegenden Erfindung näher erläutert. Es zeigt
- Figur 1-: die schematische Anordnung zweier Lagen aus einem Linermaterial,
- Figur 2 -: eine Anordnung der Mehrzahl von Fasern in einer ersten Ausrichtung,
- Figur 3 -: die Anordnung der Mehrzahl von Fasern in einer zweiten Ausrichtung,
- Figur 4 -: die schematische Darstellung eines Liners gemäß einem Ausführungsbeispiel der vorliegenden Erfindung,
- Figur 5 -: eine weitere mögliche Anordnung der Fasern in einem Liner,
- Figur 6 -: die schematische Darstellung eines rutschhemmenden Mittels,
- Figur 7 -: das rutschhemmende Mittel aus Figur 6 im angelegten Zustand in einer Schnittdarstellung,
- Figur 8 -: einen vergrößerten Ausschnitt aus Figur 7;
- Figur 9 -: eine schematische Schnittdarstellung durch eine Prothese gemäß einem weiteren Ausführungsbeispiel der vorliegenden Erfindung,
- Figur 10 -: einen vergrößerten Ausschnitt aus Figur 9,
- Figur 11 -: die schematische Schnittdarstellung durch eine weitere Prothese gemäß einem Ausführungsbeispiel der vorliegenden Erfindung,
- Figur 12-: die schematische Schnittdarstellung durch eine weitere Prothese gemäß einem Ausführungsbeispiel der vorliegenden Erfindung.

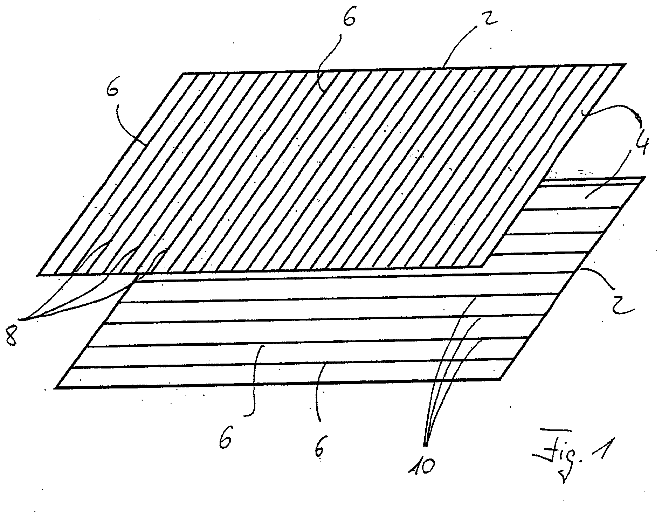

Figur 1 zeigt schematisch zwei Lagen 2 aus einem Linermaterial 4. Jede Lage 2 verfügt über eine Mehrzahl von Fasern 6, die in der jeweiligen Lage 2 parallel zueinander angeordnet sind. Zwischen den einzelnen Lagen 2 sind die Fasern 6 jedoch um 90 Grad gegeneinander versetzt. Die Gesamtheit der Fasern 6 in der oberen Lage 2 ist dabei eine erste Faserlage 8, während die Gesamtheit der Fasern 6 in der unteren Lage 2 eine zweite Faserlage 10 bildet. Im angelegten Zustand eines Liners, der die beiden Lagen 2 umfasst, führt diese Anordnung der Fasern 6 in der ersten Faserlage 8 und der zweiten Faserlage 10 dazu, dass eine elastische Verformung des Liners in einer Richtung eine elastische Verformung in einer senkrecht dazu verlaufenden zweiten Richtung zur Folge hat.

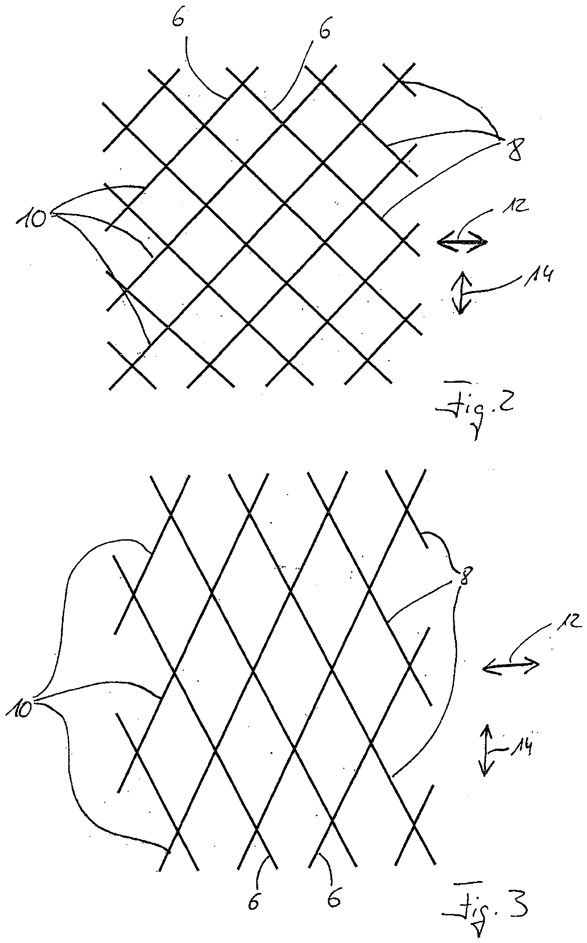

Figur 2 zeigt die erste Faserlage 8 sowie die zweite Faserlage 10 in einem Liner 16 gemäß einem Ausführungsbeispiel der vorliegenden Erfindung. Dabei ist es unerheblich, ob die erste Faserlage 8 und die zweite Faserlage 10 in einer Lage 2 oder in zwei separaten Lagen 2 angeordnet sind, die zu einem Liner übereinander gezogen werden. Man erkennt, dass die Fasern 6 der ersten Faserlage 8 und die Fasern 6 der zweiten Faserlage 10 jeweils einen rechten Winkel miteinander einschließen. Eine Verlängerung des Liners in einer ersten Richtung, die durch den Pfeil 12 in Figur 2 dargestellt ist, hat folglich eine Verkürzung des Liners 16 in einer senkrecht dazu verlaufenden zweiten Richtung, die durch den Pfeil 14 dargestellt ist, zur Folge. Dies kann insbesondere dadurch erreicht werden, dass die einzelnen Fasern 6 unelastisch oder zumindest weniger elastisch als das Linermaterial 4 ausgebildet werden, in dem sie eingebettet sind. Es ist somit nicht oder nur in sehr geringem Maße möglich, die einzelnen Fasern 6 entlang ihrer Längserstreckung zu dehnen, so dass eine Verlängerung des Liners entlang der ersten Richtung nicht oder nur in sehr geringem Maße dadurch erreicht werden kann, dass die einzelnen Fasern 6 gedehnt werden.

Figur 3 zeigt die erste Faserlage 8 und die zweite Faserlage 10 aus Figur 2, wobei der Liner nun in Richtung der zweiten Richtung, die durch den Pfeil 14 dargestellt wird, gestreckt wurde. Man erkennt, dass die Fasern 6 der ersten Faserlage 8 und der zweiten Faserlage 10 keinen rechten Winkel mehr einschließen, sondern dass nun zwischen den einzelnen Fasern gestreckte Rauten gebildeten werden. Die Ausdehnung und Verlängerung des Liners entlang der ersten Richtung des Pfeils 14 hat gleichzeitig eine Verkürzung des Liners in der dazu entgegen gesetzten zweiten Richtung, die durch den Pfeil 12 dargestellt wird, zur Folge. In einem angelegten Zustand des Liners 16 kann eine derartige Verkürzung nur erfolgen, wenn der Liner großflächig am Amputationsstumpf entlang gleitet. Da jedoch die Haftung zwischen Linermaterial 4 und Amputationsstumpf relativ hoch ist, ist ein derartiges Entlanggleiten nicht möglich. Folglich kann der Liner 16 im einmal angelegten Zustand einer Volumenänderung des Amputationsstumpfes nicht oder nur in sehr geringem Maße folgen, so dass die Volumenstabilität gegeben ist.

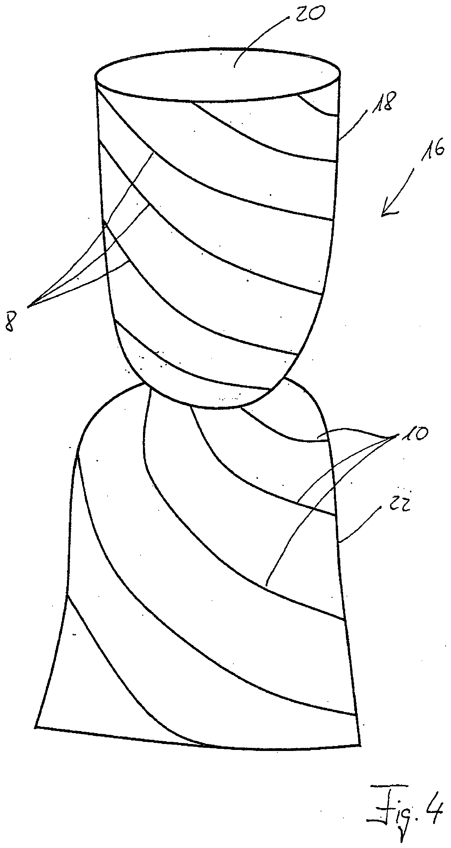

Figur 4 zeigt eine besondere Ausgestaltung eines Liners 16. Er besteht aus einem ersten Teilliner 18, der an seinem proximalen Ende eine Öffnung 20 aufweist. In diese wird der Amputationsstumpf eingeführt. Der erste Teilliner 18 verfügt über die erste Faserlage 8, die in diesem Fall eine spiralförmige Matrix bildet, die eine erste Umlaufrichtung aufweist.
Am distalen Ende des ersten Teilliners 18 ist ein zweiter Teilliner 22 angeordnet, in dem sich die zweite Faserlage 10 befindet. Auch diese ist eine spiralförmige Matrix, die jedoch in entgegengesetzter Richtung umläuft. Der zweite Teilliner 22 wird zum Anlegen des Liners 16 an einen nicht gezeigten Amputationsstumpf über den ersten Teilliner 18 abgerollt, so dass sowohl der erste Teilliner 18 als auch der zweite Teilliner 22 den Amputationsstumpf umhüllen und die erste Faserlage 8 und die zweite Faserlage 10 übereinander angeordnet werden. Dadurch, dass beide Faserlagen 8, 10 eine spiralförmige Matrix bilden, die jedoch unterschiedliche Umlaufrichtungen aufweisen, bilden die einzelnen Fasern 6 der beiden Faserlagen 8, 10 insgesamt eine netzartige Struktur, die genau die gewünschten Eigenschaften aufweist.

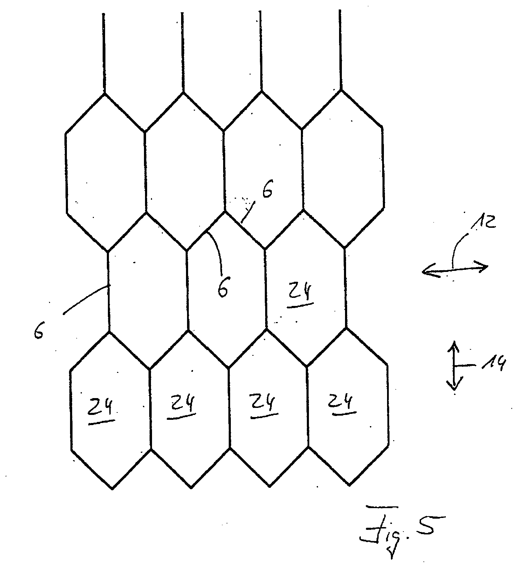

Figur 5 zeigt eine weitere Anordnungsmöglichkeit der unterschiedlichen Fasern 6, die in diesem Fall eine netzartige Struktur mit aneinander angrenzenden Maschen 24 bilden. Jeder dieser Maschen 24 weist im in Figur 5 gezeigten Ausführungsbeispiel eine hexagonale Form auf. Durch diese Form der netzartigen Struktur hat auch bei dieser Anordnung der Fasern eine Verlängerung des Liners entlang der Richtung des Pfeils 12 eine Verkürzung des Liners entlang der Richtung des Pfeils 14 und umgekehrt zur Folge, so dass auch diese Anordnung den gewünschten und erfindungsgemäßen Effekt aufweist.

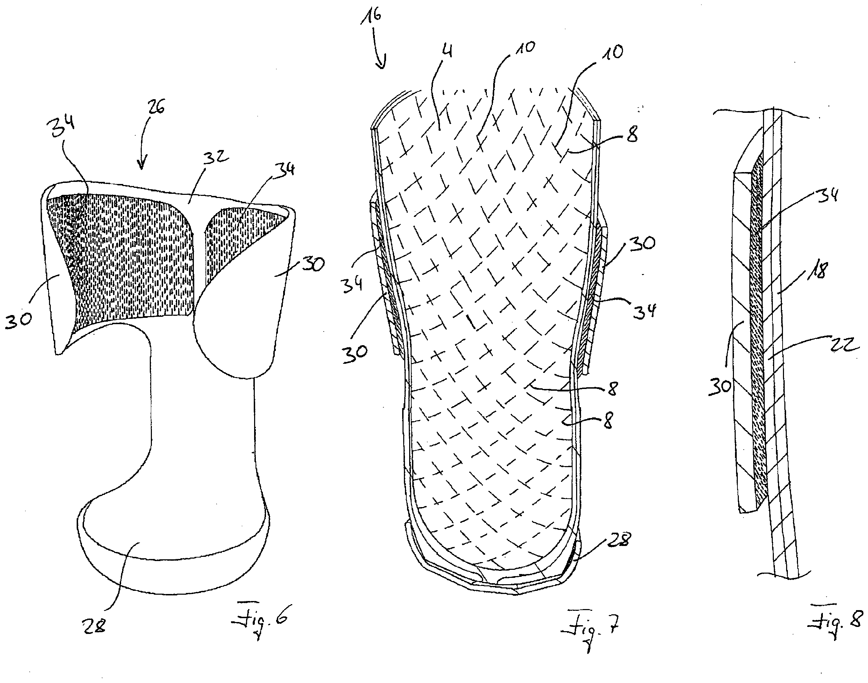

Figur 6 zeigt ein rutschhemmendes Mittel 26, wie es bei einer Prothese gemäß einem Ausführungsbeispiel der vorliegenden Erfindung verwendet werden kann. Das rutschhemmende Mittel 26 ist als separates Bauteil ausgebildet, wird aber dennoch als Teil des Liners gewertet. Es umfasst in seinem distalen Bereich eine Aufnahmetasse 28, in die das distale Ende des Amputationsstumpfes eingesetzt wird. Im proximalen Bereich des rutschhemmenden Mittels 26 sind zwei laschenartige Vorsprünge 30 angeordnet, die den Amputationsstumpf mit dem sich darum befindlichen Linermaterial zumindest teilweise umschließen. An einer Innenseite 32 sind Steigfelle 34 angeordnet, durch die eine Relativbewegung des Linermaterials zu den laschenartigen Vorsprüngen 30 zumindest in eine Richtung verhindert wird.

Figur 7 zeigt eine Schnittdarstellung durch einen Liner 16 mit einem rutschhemmenden Mittel 26. Man erkennt in dem Linermaterial 4 angeordnet die erste Faserlage 8 und die zweite Faserlage 10, die so angeordnet sind, dass sich die einzelnen Fasern kreuzen. Im distalen Bereich ist die Aufnahmetasse 28 zu erkennen, während im proximalen Bereich des rutschhemmenden Mittels 26 die beiden laschenartigen Vorsprünge 30 dargestellt sind, wobei sich an deren Innenseite 32 die Steigfelle 34 befinden. Dieser Teil ist in Figur 8 vergrößert dargestellt. Man erkennt den ersten Teilliner 18 sowie den zweiten Teilliner 22, die aneinander anliegen. An der Außenseite des zweiten Teilliners 22 befindet sich ein laschenartiger Vorsprung 30, mit dem an seiner Innenseite angeordneten Steigfell 34. Bei der gezeigten Anordnung des Steigfells 34 sind die einzelnen Haare oder Borsten des Steigfells 34 von dem laschenartigen Vorsprung 30 nach unten gerichtet, so dass ein Verschieben und Verrutschen des Linermaterials 4 relativ zu den laschenartigen Vorsprüngen 30 nach unten möglich ist, während ein Verschieben des Linermaterials 4 nach oben verhindert wird. Eine derartige Anordnung ist folglich insbesondere für den Fall sinnvoll, dass erwartet wird, dass das Volumen des Amputationsstumpfes im Laufe eines Tages abnimmt. Dies hätte eine Verschiebung des Linermaterials 4 nach oben zur Folge, da eine Verringerung der Ausdehnung des Liners 16 in Umfangsrichtung eine Verlängerung in Längsrichtung zur Folge hätte. Dieses Verschieben wird jedoch durch die Steigfelle 34 an der Innenseite der laschenartigen Vorsprünge 30 verhindert.

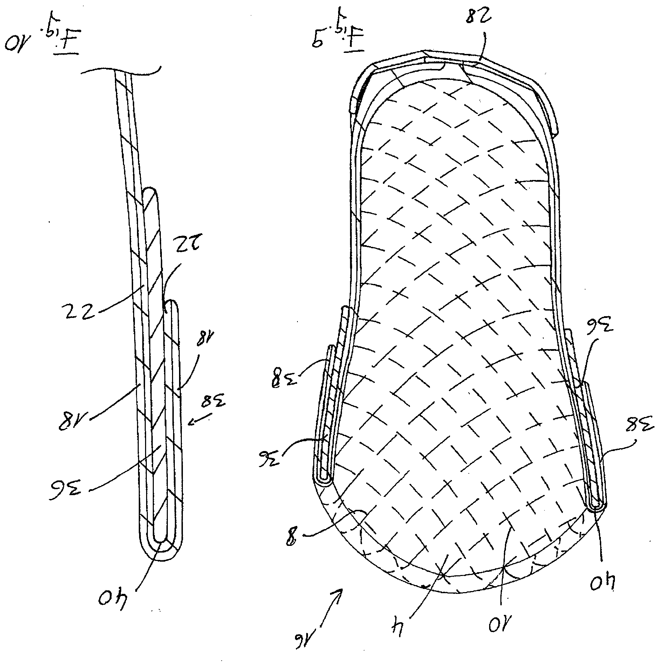

Figur 9 zeigt eine andere Ausgestaltung eines Liners 16. Auch hier sind die erste Faserlage 8 und die zweite Faserlage 10 dargestellt, die in dem Linermaterial 4 eingebettet sind. Im distalen Bereich des Liners 16 ist wieder die Aufnahmetasse 28 des rutschhemmenden Mittels angeordnet. Natürlich gibt es auch rutschhemmende Mittel ohne Aufnahmetasse 28. Im proximalen Bereich des rutschhemmenden Mittels befindet sich ein Umschließungselement 36, das im gezeigten Ausführungsbeispiel den Amputationsstumpf vollständig umgibt. Es kann beispielsweise aus einem elastischen Material gefertigt sein und wird in diesem Fall bei Anordnung des Amputationsstumpfes innerhalb des Umschließungselementes 36 gedehnt, so dass es eine Kraft in bezüglich der Längsrichtung des Amputationsstumpfes radialer Richtung aufbringt. Auf diese Weise wird in dem Bereich, in dem sich das Umschließungselement 36 befindet, eine größere Kraft auf das Linermaterial 4 und damit auch auf den sich darin befindenden Amputationsstumpf aufgebracht, so dass eine erhöhte Haftreibung erzielt wird.

Man erkennt in Figur 10, dass ein proximaler Bereich 38 des Linermaterials 4 um eine Oberkante 40 des Umschließungselementes 36 umgeschlagen ist. Diese Situation ist in Figur 10 vergrößert dargestellt. Das Umschlagen des proximalen Bereichs 38 des ersten Teilliners 18 und des zweiten Teilliners 22 hat mehrere Effekte. Zum einen wird das Umschließungselement 36 zwischen zwei Lagen des Linermaterials eingeschlossen. Dies ist insbesondere für den Fall sinnvoll, dass das rutschhemmende Mittel 26 ausschließlich aus dem Umschließungselement 36 besteht und nicht noch eine Aufnahmetasse 28 oder ein sonstiges Element aufweist, durch das eine optimale und feststehende Positionierung des Umschließungsmittels 38 am Amputationsstumpf gewährleistet wird. Zudem wird durch den umgeschlagenen proximalen Bereich 38 des Linermaterials 4 eine weitere Kraft in radialer Richtung auf das Umschließungselement und damit auch auf den sich darin befindenden Amputationsstumpf ausgeübt, so dass eine weitere Erhöhung der Haftreibung erreicht wird.

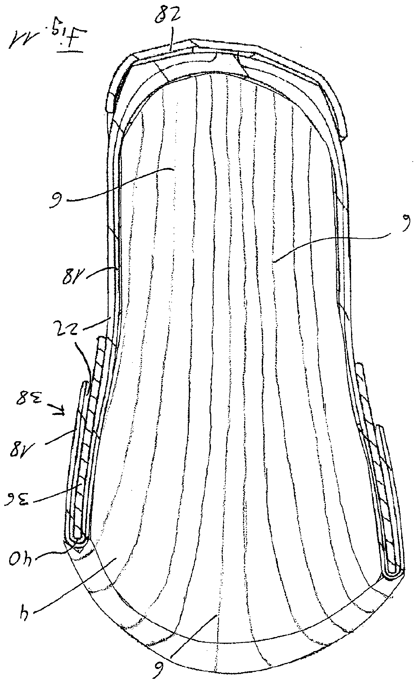

Figur 11 zeigt eine ähnliche Darstellung wie Figur 9, bei der ebenfalls ein Umschließungselement 36 vorgesehen ist, das um einen nicht gezeigten Amputationsstumpf herum gelegt ist. Um die Oberkante 40 des Umschließungselementes 36 ist ein proximaler Bereich 38 des Linermaterials 4 umgeklappt. Anders als der in Figur 9 gezeigte Liner verfügt der in Figur 11 gezeigte Liner nur über eine Faserlage, die aus Fasern 6 besteht, die sich ausschließlich in Längsrichtung des Liners, also von dem distalen Ende des Liners zum proximalen Rand des Liners erstrecken. Dies hat zur Folge, dass eine elastische Verformung in Längsrichtung nicht mehr möglich ist, obwohl sich der Liner beim Aufziehen und Abrollen auf einem Amputationsstumpf optimal der Form des Amputationsstumpfes anpassen kann.

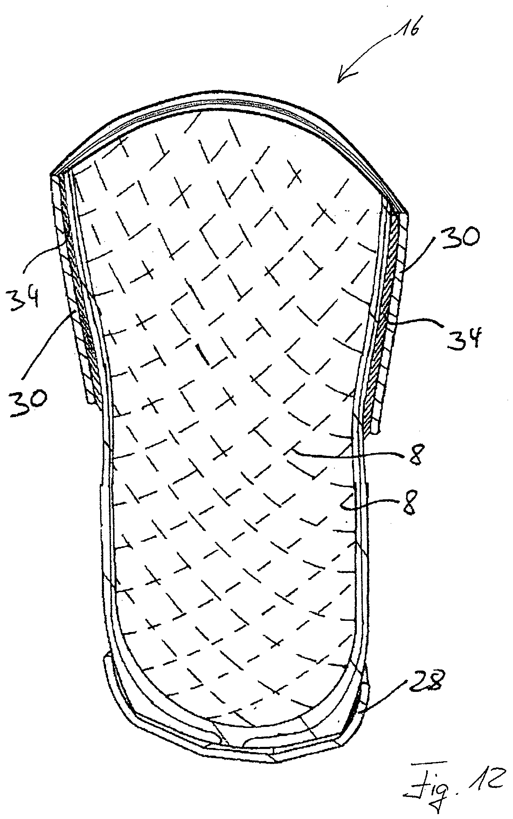

Figur 12 zeigt eine zu Figur 7 ähnliche Schnittdarstellung durch einen Liner 16 mit einem rutschhemmenden Mittel 26. Anders als der in Figur 7 gezeigte Liner 16 endet der Liner im proximalen Bereich, in Figur 12 also oben, bündig mit den laschenartigen Vorsprüngen 30 des rutschhemmenden Mittels 26. Während der in Figur 7 gezeigte Liner 16 wie in Figur 9 dargestellt umgeschlagen werden kann, und so das rutschhemmende Mittel 26 beidseitig, also von innen und von außen, zumindest teilweise umfasst, ist dies bei einem Liner 16 gemäß der in Figur 12 gezeigten Ausführungsform nicht möglich. Auch hier sind jedoch an der Innenseite 32 des rutschhemmenden Mittels 26 Steigfelle 34 angeordnet, durch die eine Relativbewegung des Linermaterials zu den laschenartigen Vorsprüngen 30 zumindest in eine Richtung verhindert wird. Für die meisten Anwendungen ist es ausreichend, die Steigfelle 34 als einziges Bauteil vorzusehen, die den Effekt der rutschhemmenden Mittel 26 hervorrufen. Ein Umklappen des Liners 16 wie in Figur 9 dargestellt um weiteren Druck von der Außenseite der laschenartigen Vorsprünge 30 aufzubringen und so die Wirkung der Steigfelle 34 zu verstärken, ist von Vorteil, aber nicht nötig.

### Bezugszeichenliste

- 2: Lage
- 4: Linermaterial
- 6: Faser
- 8: Erste Faserlage
- 10: Zweite Faserlage
- 12: Pfeil
- 14: Pfeil
- 16: Liner
- 18: Erster Teilliner
- 20: Öffnung
- 22: Zweiter Teilliner
- 24: Masche
- 26: Rutschhemmendes Mittel
- 28: Aufnahmetasse
- 30: Laschenartiger Vorsprung
- 32: Innenseite
- 34: Steigfell
- 36: Umschließungselement
- 38: Proximaler Bereich
- 40: Oberkante

## Patentansprüche

1. Liner (16) für eine Prothese, der
- eine proximale Öffnung zum Aufnehmen eines Amputationsstumpfes und
- ein der proximalen Öffnung gegenüberliegendes distales Ende aufweist und
- aus einem Linermaterial (4) hergestellt ist,
wobei sich eine Längsrichtung des Liners (16) von der proximalen Öffnung zu dem distalen Ende erstreckt und eine Umfangsrichtung senkrecht zu der Längsrichtung verläuft, wobei der Liner (16) rutschhemmende Mittel (26) aufweist, durch die ein Verrutschen des Liners (16) an dem Amputationsstumpf in einem angelegten Zustand des Liners (16) verhindert wird,
**dadurch gekennzeichnet, dass**
der Liner (16) eine Mehrzahl von Fasern (6) aufweist, die
- aus einem unelastischen Material bestehen und
- derart angeordnet sind, dass eine Verlängerung des Liners (16) in Längsrichtung eine Verkürzung des Liners (16) in Umfangsrichtung und umgekehrt zur Folge hat.

2. Liner (16) nach Anspruch 1, **dadurch gekennzeichnet, dass** die rutschhemmenden Mittel (26) eine Oberflächenbeschaffenheit einer Innerseite des Liners (16) sind, die in dem angelegten Zustand des Liners (16) an dem Amputationsstumpf anliegt.

3. Liner (16) nach Anspruch 1 oder 2, **dadurch gekennzeichnet, dass** die rutschhemmenden Mittel (26) wenigstens ein Umschließungselement (36) aufweisen, das eingerichtet ist, den Amputationsstumpf im angelegten Zustand des Liners (16) so zu umschließen, dass ein Verrutschen des Liners (16) verhindert wird.

4. Liner (16) nach Anspruch 1, 2 oder 3, **dadurch gekennzeichnet, dass** die Mehrzahl von Fasern (6) wenigstens eine erste Faser und wenigstens eine zweite Faser aufweist, die derart angeordnet sind, dass sie sich gegenseitig vorzugsweise unter einem rechten Winkel kreuzen.

5. Liner (16) nach Anspruch 4, **dadurch gekennzeichnet, dass** die erste Faser eine spiralförmige Matrix in einer ersten Umlaufrichtung bildet und die zweite Faser eine spiralförmige Matrix in einer der ersten Umlaufrichtung entgegengesetzten zweiten Umlaufrichtung bildet.

6. Liner (16) nach Anspruch 4, **dadurch gekennzeichnet, dass** die erste Faser Teil einer ersten Faserlage (8) aus einer Mehrzahl von ersten Fasern und die zweite Faser Teil einer zweiten Faserlage (10) aus einer Mehrzahl zweiter Fasern ist.

7. Liner (16) nach Anspruch 4, 5 oder 6, **dadurch gekennzeichnet, dass** der Liner wenigstens zwei Teilliner (18, 22) aufweist, wobei die wenigstens eine erste Faser in einem ersten Teilliner (18) und die wenigstens eine zweite Faser in einem zweiten Teilliner (22) eingebettet ist und der zweite Teilliner (22) vorgesehen ist, über einen Amputationsstumpf gezogen zu werden, nachdem über den Amputationsstumpf der erste Teilliner (18) gezogen wurde.

8. Liner (16) nach Anspruch 1, 2 oder 3, **dadurch gekennzeichnet, dass** die Fasern (6) sich in Längsrichtung des Liners von dem distalen Ende zu der proximalen Öffnung erstrecken.

9. Liner (16) nach Anspruch 1, 2 oder 3, **dadurch gekennzeichnet, dass** die Mehrzahl von Fasern (6) eine netzartige Struktur mit einer Mehrzahl aneinander angrenzender Maschen (24) bildet.

10. Liner (16) nach Anspruch 9, **dadurch gekennzeichnet, dass** die Maschen (24) eine hexagonale Form aufweisen.

11. Prothese mit einem Liner (16) nach einem der vorstehenden Ansprüche, einem Prothesenschaft und einer Protheseneinrichtung, die an einem distalen und/oder proximalen Ende des Prothesenschaftes festlegbar ist.

12. Prothese nach Anspruch 11, **dadurch gekennzeichnet, dass** der Prothesenschaft einen proximalen Schaftbereich und einen distalen Schaftbereich aufweist, die durch eine Verbindungseinrichtung voneinander beabstandet miteinander verbunden sind.

13. Prothese nach Anspruch 11 oder 12, **dadurch gekennzeichnet, dass** der proximale Schaftbereich eine Öffnung zum Aufnehmen eines Amputationsstumpfes aufweist, wobei eine Umfangslänge der Öffnung einstellbar ist.

## Claims

1. A liner (16) for a prosthesis, said liner (16)
- having a proximal opening for receiving an amputation stump and
- a distal end opposite the proximal opening, and
- being produced from a liner material (4),
wherein a longitudinal direction of the liner (16) extends from the proximal opening to the distal end and a circumferential direction extends perpendicularly to the longitudinal direction, wherein the liner (16) has anti-slip means (26) by way of which the liner (16) is prevented from slipping on the amputation stump in the fitted state of the liner (16),
**characterized in that**
the liner (16) has a plurality of fibers (6) which
- consist of a nonelastic material and
- are arranged such that an elongation of the liner (16) in the longitudinal direction results in shortening of the liner (16) in the circumferential direction and vice versa.

2. The liner (16) as claimed in claim 1, **characterized in that** the anti-slip means (26) are a surface property of an inner side of the liner (16), said inner side resting against the amputation stump in the fitted state of the liner (16).

3. The liner (16) as claimed in claim 1 or 2, **characterized in that** the anti-slip means (26) have at least one enclosing element (36) which is designed to enclose the amputation stump in the fitted state of the liner (16) such that the liner (16) is prevented from slipping.

4. The liner (16) as claimed in claim 1, 2 or 3, **characterized in that** the plurality of fibers (6) has at least a first fiber and at least a second fiber which are arranged such that they cross one another preferably at a right angle.

5. The liner (16) as claimed in claim 4, **characterized in that** the first fiber forms a spiral matrix in a first circumferential direction and the second fiber forms a spiral matrix in a second circumferential direction opposite to the first circumferential direction.

6. The liner (16) as claimed in claim 4, **characterized in that** the first fiber is part of a first fiber layer (8) made of a plurality of first fibers and the second fiber is part of a second fiber layer (10) made of a plurality of second fibers.

7. The liner (16) as claimed in claim 4, 5 or 6, **characterized in that** the liner has at least two part-liners (18, 22), wherein the at least one first fiber is embedded in a first part-liner (18) and the at least one second fiber is embedded in a second part-liner (22), and the second part-liner (22) is intended to be pulled over an amputation stump after the first part-liner (18) has been pulled over the amputation stump.

8. The liner (16) as claimed in claim 1, 2 or 3, **characterized in that** the fibers (6) extend in the longitudinal direction of the liner from the distal end to the proximal opening.

9. The liner (16) as claimed in claim 1, 2 or 3, **characterized in that** the plurality of fibers (6) forms a net-like structure having a plurality of mutually adjoining meshes (24).

10. The liner (16) as claimed in claim 9, **characterized in that** the meshes (24) have a hexagonal shape.

11. A prosthesis having a liner (16) as claimed in one of the preceding claims, a prosthesis socket and a prosthetic device which is securable to a distal and/or proximal end of the prosthesis socket.

12. The prosthesis as claimed in claim 11, **characterized in that** the prosthesis socket has a proximal socket region and a distal socket region which are connected together in a spaced-apart manner by a connecting device.

13. The prosthesis as claimed in claim 11 or 12, **characterized in that** the proximal socket region has an opening for receiving an amputation stump, wherein a circumferential length of the opening is settable.

## Revendications

1. Doublure (16) pour une prothèse, qui
- présente une ouverture proximale pour recevoir un moignon d'amputation, et
- présente une extrémité distale opposée à l'ouverture proximale, et
- est réalisée en un matériau de doublure (4),
une direction longitudinale de la doublure (16) s'étendant depuis l'ouverture proximale jusqu'à l'extrémité distale, et une direction périphérique s'étendant perpendiculairement à la direction longitudinale, la doublure (16) présentant des moyens anti-glissants (26) permettant d'empêcher un glissement de la doublure (16) sur le moignon d'amputation dans un état appliqué de la doublure (16),
**caractérisée en ce que**
la doublure (16) comprend une multitude de fibres (6) qui
- sont constituées en un matériau inélastique et qui
- sont disposées de telle sorte qu'un allongement de la doublure (16) en direction longitudinale provoque un raccourcissement de la doublure (16) en direction périphérique, et inversement.

2. Doublure (16) selon la revendication 1,
**caractérisée en ce que**
les moyens anti-glissants (26) sont une structure de surface d'une face intérieure de la doublure (16), qui est en appui contre le moignon d'amputation, dans l'état appliqué de la doublure (16).

3. Doublure (16) selon la revendication 1 ou 2,
**caractérisée en ce que**
les moyens anti-glissants (26) présentent au moins un élément d'entourage (36) qui est conçu pour entourer le moignon d'amputation, dans l'état appliqué de la doublure (16), de manière à empêcher un glissement de la doublure (16).

4. Doublure (16) selon la revendication 1, 2 ou 3,
**caractérisée en ce que**
la multitude de fibres (6) comprend au moins une première fibre et au moins une seconde fibre qui sont disposées de manière à se croiser mutuellement de préférence à angle droit.

5. Doublure (16) selon la revendication 4,
**caractérisée en ce que**
la première fibre forme une matrice spiralée dans une première direction périphérique, et la seconde fibre forme une matrice spiralée dans une seconde direction périphérique opposée à la première direction périphérique.

6. Doublure (16) selon la revendication 4,
**caractérisée en ce que**
la première fibre fait partie d'une première couche de fibres (8) constituée d'une multitude de premières fibres, et la seconde fibre fait partie d'une seconde couches de fibres (10) constituée d'une multitude de secondes fibres.

7. Doublure (16) selon la revendication 4, 5 ou 6,
**caractérisée en ce que**
la doublure comprend au moins deux doublures partielles (18, 22), ladite au moins une première fibre étant noyée dans la première doublure partielle (18) et ladite au moins une seconde fibre étant noyée dans une seconde doublure partielle (22), et la seconde doublure partielle (22) est prévue pour passer par-dessus un moignon d'amputation une fois que la première doublure partielle (18) a été passée par-dessus le moignon d'amputation.

8. Doublure (16) selon la revendication 1, 2 ou 3,
**caractérisée en ce que**
les fibres (6) s'étendent en direction longitudinale de la doublure depuis l'extrémité distale jusqu'à l'ouverture proximale.

9. Doublure (16) selon la revendication 1, 2 ou 3,
**caractérisée en ce que**
la multitude de fibres (6) forme une structure en forme de réseau ayant une multitude de mailles (24) adjacentes les unes aux autres.

10. Doublure (16) selon la revendication 9,
**caractérisée en ce que**
les mailles (24) présentent une forme hexagonale.

11. Prothèse comportant une doublure (16) selon l'une des revendications précédentes, une emboîture de prothèse et un dispositif prothétique qui peut être immobilisé sur une extrémité distale et/ou proximale de l'emboîture de prothèse.

12. Prothèse selon la revendication 11,
**caractérisée en ce que**
l'emboîture de prothèse présente une zone d'emboîture proximale et une zone d'emboîture distale qui sont reliées l'une à l'autre en étant espacées l'une de l'autre par un moyen de liaison.

13. Prothèse selon la revendication 11 ou 12,
**caractérisée en ce que**
la zone d'emboîture proximale présente une ouverture pour recevoir un moignon d'amputation, une longueur périphérique de l'ouverture étant réglable.
